# EUROPEAN PATENT APPLICATION

(11) **EP 4 167 254 A1**
(43) Date of publication of application: **19.04.2023**
(21) Application number: 21202190.1
(22) Date of filing: 12.10.2021
(51) Int. Cl.: H01F 1/00, H01F 1/06, G01N 33/543, G01N 33/68

(54) **FUNCTIONALIZED MAGNETS AND METHODS OF PRODUCING THEM**

(71) Applicant: PreOmics GmbH, 82152 Planegg/Martinsried (DE)
(72) Inventor: The designation of the inventor has not yet been filed
(74) Representative: Dentons Patent Solutions Rechtsanwaltsgesellschaft mbH

(57) **Abstract**

The present invention relates to a solid, magnetized or magnetizable composition comprising (i) a core consisting of or comprising a ferromagnetic or ferrimagnetic material; and (ii) at least one surface moiety selected from (1) a binding moiety; (2) a biomolecule; and (3) a chemically reactive moiety; wherein said moiety is covalently bound to (a) said core; or (b) a layer which is directly or indirectly attached to said core. The invention furthermore provides methods of producing such compositions and uses thereof.

## Description

The present invention relates to a solid, magnetized or magnetizable composition comprising (i) a core consisting of or comprising a ferromagnetic or ferrimagnetic material; and (ii) at least one surface moiety selected from (1) a binding moiety; (2) a biomolecule; and (3) a chemically reactive moiety; wherein said moiety is covalently bound to (a) said core; or (b) a layer which is directly or indirectly attached to said core.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Functionalized paramagnetic microparticles are a commonly used means for enriching analytes comprised in complex samples. To that end, the particles typically carry moieties on their surface which are capable of binding cognate binding partners. The present inventors recognized earlier that using ferromagnetic or ferrimagnetic material instead of paramagnetic ones entails distinct advantages as does the use of a small number or only one macroscopic magnet as opposed to a large number of microparticles. In particular, a macroscopic magnet, when under the influence of a fluctuating magnetic field, turned out to be capable of lysing cellular material and viruses, and of fragmenting biomolecules, thereby dispensing with the need for enzymes of biological origin in workflows of biological or clinical sample preparation for downstream analytical methods such as mass spectrometry (MS); see EP 19733052.5, PCT/EP2021/062677, PCT/EP2021/062680, and PCT/EP2021/062681.

These new developments of the present inventors entail the need for functionalized macroscopic magnets in the context of further optimized workflows. Their production is a non-trivial exercise. In particular, and aiming at efficient production, a plurality of magnets would have to be exposed to conditions which allow for their functionalization. The strong magnetic interaction between magnets, however, interferes with the functionalization process and renders the surface coverage with the desired functional moieties difficult or inhomogeneous.

The present application addresses inter alia the problem of manufacturing functionalized magnets. By providing corresponding processes as disclosed further below, the present application furthermore addresses the problem of providing functionalized magnets themselves, which, as stated above, are useful for various aspects of sample preparation.

The solutions to these problems are subject-matter of this invention.

In particular, and in a first aspect, the invention provides a solid, magnetized or magnetizable composition comprising (i) a core consisting of or comprising a ferromagnetic or ferrimagnetic material; and (ii) at least one surface moiety selected from (1) a binding moiety; (2) a biomolecule; and (3) a chemically reactive moiety; wherein said moiety is covalently bound to (a) said core; or (b) a layer which is directly or indirectly attached to said core.

The term "core" defines a single block of ferro- or ferrimagnetic material. Both the core and functionalized versions thereof are also referred to as "particle" herein. Of note, though, a particle of the invention is not be confused with paramagnetic particles. In particular, the core is ferro- or ferrimagnetic which renders it permanently magnetized (after magnetization has occurred) while paramagnetic particles only respond to external magnetic fields. Moreover, the core of the invention is larger as compared to paramagnetic particles which generally are of a size in the sub-µm to µm range.

Useful materials in this respect are known in the art and disclosed further below. Of note, such materials require a process of magnetization in order to convert them into a magnets.

For that reason, this disclosure distinguishes between "magnetizable" and "magnetized", wherein the former refers to the capability of being converted into a magnet (which conversion has not yet occurred), and the latter refers to the result of the process of magnetization. The magnetized composition of the invention is also referred to as "magnet" herein, or, given that it has moieties or molecules bound to its surface, as "functionalized magnet".

"Magnetic" as used in "ferromagnetic" and "ferrimagnetic" has its art-established meaning and refers to a material which is magnetizable without specifying whether it has actually been magnetized or not. In other words, "magnetic" embraces both "magnetizable" and "magnetized".

Since the core is a single block of material, it is understood that also the composition of the first aspect is a single block - to be distinguished from a powder which is not within the ambit of the present invention. Said core, said block and said composition are furthermore solid, wherein "solid" refers to one of the basic states of matter, to be distinguished from fluid and gaseous states.

The core may entirely consist of said magnetic material, but may also comprise it, e.g. in dispersed form within an - also solid - matrix material. Furthermore, it may occur that the magnetic material is not entirely pure - also in such a case the core may be viewed as comprising magnetic material. Preferred is pure or essentially pure magnetic material.

What is generally referred to as "functionalized" in the introductory part herein above, is called out more specifically in the first aspect, i.e., binding moieties, biomolecules, and chemically reactive moieties being covalently attached. More preferred implementations of functionalized magnets are disclosed further below.

Said moieties or molecules are located on the surface of the magnet or the magnetizable composition. As consequence, said moieties or molecules are exposed and available for interaction when brought into contact with a sample, generally a liquid sample.

The recitation of "at least one surface moiety" embraces the scenario of a single molecule, such as a polymer, covering the entire magnet or composition. It also refers to the more common scenario of a multitude of moieties being bound to the surface of a single magnet or composition.

The density of molecules is not particularly limited and may be chosen such that constituents of sample brought into contact with the composition have contact only or essentially only said moieties. On the other, it may be chosen such that constituents of the sample can access, to a certain degree, the surface of the core to which said moieties are attached.

In terms of attachment, as specified in the first aspect, this may either be direct in which case the moieties are covalently bound to the magnetic material itself. On the other hand, this may be indirect - an embodiment which is defined by the terms "indirect" and "layer".

A layer is a thin coat which preferably covers the entire core. Albeit less preferred, it is also envisaged that said layer is a patch, i.e., it covers only a certain fraction of the surface of the core.

In case a layer is present, the covalent attachment of said moieties is to said layer.

Advantages of the compositions and magnets of the invention are apparent from the methods and uses which are subject of the fourth and further aspects as disclosed further below. Related thereto, and as mentioned above, the magnets of the present invention confer distinct advantages in the context of applicant's earlier applications EP 19733052.5, PCT/EP2021/062677, PCT/EP2021/062680, and PCT/EP2021/062681. To explain further, the functionalized magnets of the present invention are capable of performing cell lysis and biomolecule fragmentation as described in these earlier applications, and furthermore binding including specific binding in view their functionalization with the moieties defined herein.

In terms of embodiments of the composition of the first aspect, it is preferred that said layer is (a) the only layer and is directly attached to said core, preferably plated, coated or melted onto said core; or (b) an outermost layer which is indirectly attached to said core and separated therefrom by one or more further layers, wherein adjacent layers are directly attached to each other, and wherein an innermost layer is directly attached to said core, preferably plated, coated or melted onto said core.

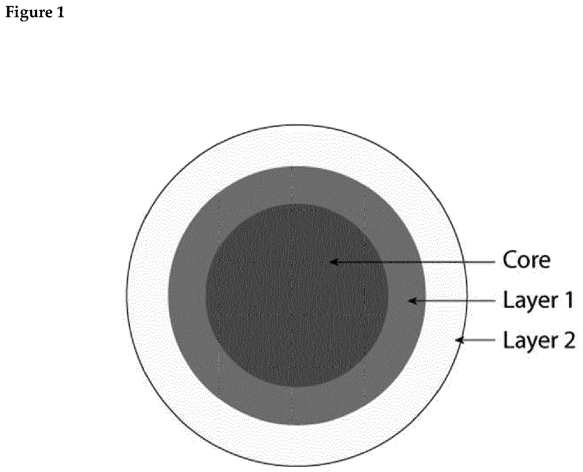

This embodiment provides for two or more layers. Such layers are placed on top of each other; see, e.g. Figure 1. The term "adjacent" refers to a pair of layers with one being bound to and on top of the other one. For clarity, the terms "outermost" and "innermost" layer have been introduced. It is the outermost layer to which said moieties are covalently bound. The innermost layer is directly attached to the core which comprises or consists of magnetic material. All layers together form something akin to a multitude of onion skins which are bound to each other with the core in their center.

Between the core and the innermost layer as well as between layers there is no spacing, regardless of whether such spacing would be filled with vacuum or any, possibly gaseous or liquid material. In other words, the innermost layer is bound to the core and layers are bound to each other. The term "bound" refers to any interaction between atoms and molecules and includes van der Waals interactions, hydrophobic interactions, chelating interactions, stacking interactions, interactions involving permanent and/or fluctuating dipoles as well as covalent interactions. As a consequence, when it is stated above that the outermost layer is "separated" from the core, this refers to a certain distance from the core which is owed to the intervening layer(s). It does not mean that there would an empty space.

In particular as regards the innermost layer, it is of note that many commercially available magnets or magnetizable compositions are available with such a layer already present. Various processes for applying the innermost layer are known in the art and are referred to as plating including electroplating, coating and melting. Such processes may be applied repeatedly, thereby giving rise to an innermost layer which in turn has a layered structure.

This is referred to as "structure" of said layer in the following, and the results of the repeated application of coating, plating or melting are referred to as sub-layers herein.

In a further preferred embodiment,
(1) said binding moiety is configured for chromatography such as cation exchange chromatography (preferred moieties being sulfate and carboxylate), anion exchange chromatography (a preferred moiety being a primary, secondary, tertiary or quaternary amine), reversed-phase chromatography (preferred moieties being C18, C8, C4 and phenyl), normal-phase chromatography such as hydrophilic interaction chromatography (preferred moieties being silanol, diol, and amide), immobilized metal-ion exchange chromatography (preferred moieties being chelates of metal ions Zn²⁺, Cu²⁺, Cd²⁺, Hg²⁺, Co²⁺, Ni²⁺, Ti⁴⁺ and/or Fe2+), mixed-phase chromatography (preferred moieties being a combination of cation-exchange and reversed-phase moieties and a combination of anion-exchange and reversed-phase moieties), and affinity chomatography;
(2) said biomolecule is a protein such as an enzyme; a polypeptide; a peptide; a nucleic acid such as DNA or RNA; or an aptamer; or
(3) said chemically reactive moiety is selected from carboxyl-to-amine reactive groups, amine-reactive groups, sulfhydryl-reactive groups, aldehyde-reactive groups, photo-reactive groups and hydroxyl (non-aqueous)-reactive groups. This embodiment enumerates preferred implementations of the above introduced moieties and molecules.

Of note, the term binding moiety embraces also those biomolecules which recognize and bind a cognate ligand. Such interactions are useful for affinity chromatography.

Chemically reactive molecules or moieties are well known in the art and can be chosen by a skilled person depending on the envisaged particular application without further ado. Preferred chemically reactive moieties can be grouped as follows:
1) Carboxyl-to-amine reactive groups such as Carbodiimide (e.g., 1-Ethyl-3-(3-dimethylaminopropyl)carbodiimide (EDC))
2) Amine-reactive groups such as N-Hydroxysuccinimid (NHS) ester, Imidoester, Pentafluorophenyl ester, or Hydroxymethyl phosphine
3) Sulfhydryl-reactive groups such as Maleimide, Haloacetyl (Bromo- or Iodo-), Pyridyldisulfide, Thiosulfonate, or Vinylsulfone
4) Aldehyde-reactive groups such as oxidized sugars (carbonyls), Hydrazide, or Alkoxyamine
5) Photoreactive groups such as nonselective, random insertion Diazirine, or Aryl Azide
6) Hydroxyl (nonaqueous)-reactive groups such as Isocyanate

Further examples can be found in Bioconjugate Techniques, 2013 Elsevier Inc., ISBN 978-0-12-382239-0, https://doi.org/10.1016/C2009-0-64240-9.

Said moieties may be covalently bound to a layer or the core via a linker. In functional terms, the linker provides distance from the surface and flexibility. In structural terms, this may be accomplished by a variety of means including alkyl chains, PEG of various lengths, peptidic linkers such as short peptides rich in Gly and optionally Ser.

A preferred implementation of embodiment (b) of the first aspect provides that (a) in case of one single layer, said single layer; and (b) in case of a plurality of layers, at least one layer, preferably at least said outermost layer, consists of or comprises a material which is silica-based, preferably silica, silicates or glass; or is a polymer, preferably comprising styrene, an olefin, an acrylic moiety, an amide and/or a urethane.

Both silica-based and polymer materials may be rendered porous, either during the process of attaching them or subsequent thereto. In particular for the outermost layer it is preferred that it is porous.

In case of a composition or magnet with multiple layers, two adjacent layers may be of the same material, wherein one of the layers may be the outermost layer and is rendered porous. For example, moieties may be covalently bound to a porous silica layer, wherein said porous silica layer is on top of a non-porous silica layer which in turn is bound to an innermost layer covering directly the core. For other preferred layer implementations, in particular of the innermost layer, see the following embodiment.

Another preferred implementation of embodiment (b) of the first aspect provides that (a) in case of one single layer, said single layer; and (b) in case of a plurality of layers, at least one layer, preferably at least said innermost layer, is a metal-containing layer which consists of or comprises at least a main group metal, a transition group metal, a compound comprising such metal, or an alloy thereof, preferably selected from Au, Zn, Zn chromate, Cu, Ni, Cr, Sn, Ti, TiN and/or Ag.

The above two preferred implementations also work in conjunction which is particularly preferred, i.e. two or more layers are present, wherein said outermost layer comprises or consists of silica-based material as defined above or polymer material as defined above, and said innermost layer is said metal-containing layer.

Said metal-containing layer preferably has a structure Xi - (Xm)k - Xo, wherein Xi is an inner sub-layer which is directly attached to said core; Xm is a middle sub-layer with k being 0, 1, 2, or 3; and Xo is an outer sub-layer, wherein preferably Xi is plated, coated or melted onto said core and/or adjacent sub-layers are plated, coated or melted onto each other, and wherein if k is greater than 1, sub-layers Xm preferably differ from each other in composition.

On the other hand, layers of Zn generally do not have such structure of multiple sub-layers.

Particularly preferred implementations of said structure are as follows:
(i) Xi - Xo, preferably Ni - Cu;
(ii) Xi - Xm - Xo, preferably Ni - Cu - Ni or Ni - Cu - Sn; or
(iii) Xi - (Xm)2 - Xo, preferably Ni -- Cu -- Ni -- Cr or Ni -- Cu -- Ni -- Au.

The thickness of the various layers is not particularly limited. Having said that, thickness will generally be in the range from about 1 µm to about 1000 µm. For example, layers of silica-based material preferably are between about 1 µm and about 100 µm such as between about 10 µm and about 60 µm. Layers of polymer material are preferably between about 100 µm to about 1000 µm such as about 200 µm. Metal layers may in the single to double digit µm range such as 1 to 20 µm or 2 to 10 µm, for example 5 µm.

As mentioned above, it is preferred that the outermost layer is porous. Preferred degrees of porosity are defined further below.

In a second aspect, the present invention provides a method of producing a solid magnetized composition, said method comprising: (a) providing a core consisting of or comprising a ferromagnetic or ferrimagnetic material, said core optionally having one or more layers as defined in any one of claims 1 to 9 attached thereto; (b) covalently attaching at least one surface moiety as defined in claim 1 or 3 to the surface of said core of (a); and (c) thereafter magnetizing the result obtained in step (b); thereby obtaining said solid magnetized composition.

Said method is suitable for the manufacture of the compositions and magnets of the first aspect. A key feature of the method is that functionalization occurs prior to magnetization. This entails distinct advantages. In particular, prior to magnetization the magnetic materials are easier to handle. Moreover, since not yet magnetized cores will not adhere to each other or to a much lesser extent compared to magnetized ones, any functionalization will be characterized by a particularly high degree of homogeneity. Yet further, not yet magnetized but functionalized compositions may be transported with less restrictions. In such a case, step (a) and step (b) would still be performed in the indicated order, but at different sites.

The precise chemical nature of said "covalently attaching" depends on both the moieties to be attached and the layer or the core to which they are to be attached. Generally speaking, useful mechanisms include addition processes such as nucleophilic addition and electrophilic addition as well as substitution processes (nucleophilic or electrophilic). In structural terms, material surfaces rich in X-H groups, X being a heteroatom such as O, S and N, are particularly apt to functionalization, i.e., the covalent attachment of moieties as defined above.

For example, base metals generally have a surface which is rich in hydroxyl groups. Silicon-based compounds bind thereto covalently because hydroxyl groups on base metal surfaces link to silicon atoms.

In case of noble metals such as Au, derivatization of Au with sulfur yields a surface chemistry which lends itself to coupling via the above-mentioned reactions; see, for example, Pensa, Evangelina & Cortes, Emiliano & Corthey, Gastón & Carro, Pilar & Vericat, Carolina & Fonticelli, Mariano & Benitez, G. & Rubert, Aldo & Salvarezza, Roberto. (2012). The Chemistry of the Sulfur-Gold Interface: In Search of a Unified Model. Accounts of chemical research. 45. 1183-92. 10.1021/ar200260p.

If a layer of silica-based material is to be functionalized with moieties as defined above, a silica-based material with silanol (Si-OH) may be used analogous to metals with hydroxyl groups on their surface. See, for example, 1. Surface Chemistry of Silicon, Hanne Neergaard Waltenburg and John Yates, Chemical Reviews 1995 95 (5), 1589-1673, DOI: 10.1021/cr00037a600.

Functionalization of Zn surfaces is described, for example in El-Nahhal et al., Synthesis & characterization of silica coated and functionalized silica coated zinc oxide nanomaterials, Powder Technology, Volume 287, January 2016, Pages 439-446.

Tosylation, in particular of hydroxyl groups, is a further well-established example of functionalization; see, for example, Velusamy et al., Cobalt(II) catalyzed tosylation of alcohols with p-toluenesulfonic acid, Tetrahedron Letters, Volume 45, Issue 1, 1 January 2004, Pages 203-205.

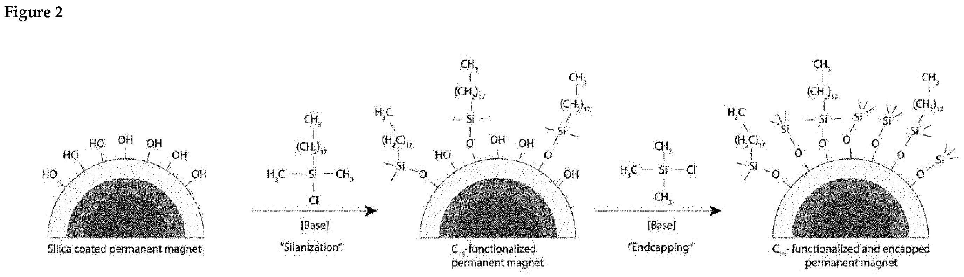

An exemplary functionalization process is described in Example 1. Figure 2 shows an exemplary workflow.

The process of magnetization is not particularly limited. It may be performed using art-established procedures. For example, temporary presence of a strong magnetic field is a means of magnetizing magnetic materials; see, e.g. Methods of Magnetizing Permanent Magnets, EMCW Coil Winding Show, 1 October-2 November 2000, Cincinnati, Ohio,Joseph J. Stupak Jr., Oersted Technology Corp.; and R. Parker and R. Studders, Permanent Magnets and their Application, Wiley 1962, and therein in particular Chapter 7, Magnetization and Demagnetization.

In a preferred embodiment of the method of the second aspect, said method further comprises a step of attaching at least one of said one or more layers as defined in any one of claims 1 to 9, wherein said further step is to be effected prior to step (b).

In a further preferred embodiment, the outermost layer of said one or more layers is rendered porous.

There are several art-established ways to render a material porous during its production or deposition. They include the use of porogens. This are elements or molecules occupying a certain amount of space which can be tailored according to the respective desired size of pores and extent of porosity. Once the porous material has set, such elements or molecule can be removed. Exemplary materials where this technique can be applied are silica-based materials such as glass, and polymers. On the other hand, porosity may be introduced after production or deposition of a layer. Suitable processes in this respect include etching, wherein etching can be effected in various ways including acids and electricity.

In a further preferred embodiment, said core has not been previously magnetized. In other words, preference is given to magnetic materials for the core which have not undergone any magnetization (and possible demagnetization) processes prior to performing the method of the second aspect.

In a further preferred embodiment, step (b) is performed repeatedly.

Such repetitions of step (b) may be performed with the same moiety or different moieties. Such same or different moieties may be attached to the same surface or the moiety introduced in the second performance of step (b) may be attached to results of the first performance of step (b), e.g. to the moiety introduced in the course of said first performance.

The fact that the involved moieties, after coupling, are attached to the magnet allows convenient handling such as separation by magnetic means of the result of a previous round of coupling from the corresponding reaction mixture prior to starting the next round.

Said magnetic means are not particularly limited. A permanent magnet outside the vessel where said brining into contact is performed may be used, wherein said permanent magnet is for example part of a device which allows moving such vessel and the external permanent magnet relative to each other. Alternatively, an electromagnet may be used which can be turned on and off.

In a preferred embodiment, said surface moiety is a first chemically reactive moiety, and wherein step (b) is repeated with a second chemically reactive moiety which is capable of reacting with said first chemically reactive moiety, thereby forming a covalent bond.

Preferred in the context of the preceding embodiment is that said second chemically reactive moiety comprises a further reactive group, wherein said further reactive group is capable of reacting with another reactive group of a further chemically reactive moiety.

This is preferably implemented such that said second moiety and said further moiety, and preferably said first moiety belong to the same class of compounds, and preferably are amino acids or nucleotides.

Generally speaking, when chemically reactive moieties are employed, in particular moieties with two reactive groups, the reactive groups or one of said reactive groups may be protected. A second reactive group within the same moiety may be de-protected once a reaction of the first reactive group has occurred.

Preferred features of both the composition of the first aspect and the method of the second aspect are as follows:
Preferably, said ferromagnetic or ferrimagnetic material is selected from
(i) compounds or alloys comprising a rare earth metal such as Nd or Sm; Fe; Co; and/or Ni; said compounds or alloys preferably being selected from NdFeB, SmCo, bismanol, AINiCo and ferrites such as Fe2O3 and Fe3O4; and
(ii) plastic magnetic material, preferably PANiCNQ.

Preferably, the size of said core is or exceeds 0.1, 0.2, 0.3, 0.4, 0.5, 0.6, 0.7, 0.8, 0.9, 1.0, 1.5, 2, 3, 4, 5, 6, 7, 8, 9 or 10 mm in at least one dimension. Preferred cores have more than about 0.5 mm, more preferred about 2 mm extension in all three dimensions.

Preferably, said core has a shape selected from sticks, bars, rods, rods with rounded ends, cubes, cuboids, prisms, spheres, elongate and oblate ellipsoids, disks, tetrahedrons, octahedrons, icosahedrons and dodecahedrons.

"Porous" preferably refers to a porosity of > 20 %; a specific surface of 40 - 500 m²/g; and/or a pore size between about 10 and about 100000 Angstrom such as about 60, about 100, about 120, about 200, about 300, about 1000, about 1500, about 2000, about 5000, about 10000, or about 50000 Angstrom.

In a third aspect, the invention provides a solid magnetized composition obtained by the method of any one of claims 10 to 21.

In a fourth aspect, the invention relates to a method of enriching or purifying an analyte comprised in a sample, said method comprising: (a) bringing a solid magnetized composition in accordance with the first aspect into contact with said sample, a. under conditions which allow binding of said analyte to said surface moiety, wherein said surface moiety is a binding moiety; and/or b. optionally changing the physicochemical conditions of said sample; (b) separating the composition with said analyte bound thereto from the remainder of said sample, wherein said separating is effected by magnetic means; and (c) optionally eluting the bound analyte from the composition.

Said conditions can be chosen depending on the specific moiety - analyte interaction of interest. Generally, such conditions will be in solution such as a buffered solution. Preferably the surface moieties on the magnet are activated and/or equilibrated prior to said bringing into contact. Such measures are well known, for example in the field of chromatography.

In addition, said conditions may be modified after said bringing into contact. Various means of modifying physicochemical conditions have been described; see, for example E. J. Cohn, L. E. Strong, W. L. Hughes, D. J. Mulford, J. N. Ashworth, M. Melin, and H. L. Taylor; Journal of the American Chemical Society 1946 68 (3), 459-475; DOI: 10.1021/ja01207a034. The present inventors furthermore found, as described in EP 21158984.1, that the presence of a suitable surface in combination with suitable physicochemical conditions can lead to a gentle precipitation of such analytes, which are otherwise not accessible with state-of-the art precipitation methods. The compositions and magnets in accordance with the present invention provide such suitable surface. In terms of physicochemical conditions, less harsh conditions than those described in Cohn et al., loc. cit. may be employed..

On the other hand, a change of physicochemical conditions may be dispensable, in particular in those cases where the moiety - analyte interaction itself provides for enrichment of the analyte on the surface of the magnet.

Suitable magnetic means are disclosed further above.

In a fifth aspect, the invention provides a use of a solid magnetized composition in accordance with the first aspect for analysis and/or purification of a sample and/or for precipitating an analyte on said composition. Such use corresponds to the method of the fourth aspect. The considerations in relation to the fourth aspect apply mutatis mutandis.

In a sixth aspect, the present invention relates to the use of a solid magnetized composition in accordance with the first aspect, wherein said surface moiety is a chemically reactive moiety, in solid phase synthesis, preferably of peptides, polypeptides or nucleic acids. Such use relates to those preferred embodiments of the method of the second aspect which pertain to chemical synthesis employing a plurality of reactive moieties. The considerations in relation to these preferred embodiments apply mutatis mutandis.

Depending on their respective activities, biomolecules allow for distinct uses of magnets which are functionalized with said biomolecules. Accordingly, in a seventh aspect, the invention provides a use of a solid magnetized composition in accordance with the first aspect, wherein said surface moiety is a biomolecule, and wherein
(i) said biomolecule is a binding molecule, for separating a cognate binding partner of said binding molecule from a sample comprising said binding partner; or
(ii) said biomolecule is an enzyme, for converting a substrate comprised in a sample into a product.

In a preferred embodiment of the method of the fourth aspect and of the uses of the fifth, sixth and seventh aspect, said method or said use furthermore comprises
(a) mixing;
(b) lysis of biological cells and/or viruses if present; and/or
(c) fragmenting biomolecules such as proteins or nucleic acids if present,
wherein any of (a) to (c) is effected by exposing said solid magnetized composition to a fluctuating magnetic field.

As mentioned in the introduction, the present inventors described in earlier patent applications EP 19733052.5, PCT/EP2021/062677, PCT/EP2021/062680, and PCT/EP2021/062681 means and methods of lysing cells and viruses as well as enzyme-free fragmenting of biomolecules which opens new avenues for sample treatment. This preferred embodiment combines these means and methods with the magnets of the present invention and their uses.

### The Figures show:

- **Figure 1:**: Exemplary structure (cross-section) of a composition of the invention.
- **Figure 2:**: Exemplary reaction scheme for preparing compositions of the invention.

The Examples illustrate the invention.

### Example 1

### Preparation of a functionalized magnet

A spheric particle consisting of Nd₂Fe₁₄B₂ with a diameter of 2 mm and a zinc coating with a thickness of around 5 µm is used for the process. The particle is not yet magnetized.

To prepare the zinc coating for further functionalization, it needs to be oxidized to ZnO first. This is done by incubating the magnet in a Cu(II)SO₄ solution until the coating of the magnet turns dark.

The zinc oxide layer is functionalized through a sol-gel process. Briefly, ten particles are added to a mixture of 40 mL ethanol, 10 mL deionized water and 1.2 mL of 28 wt.% concentrated ammonia solution and ultrasonicated for 1 h. To the solution 0.45 mL of tetraethylorthosilicate (TEOS) is added dropwise. After mixing for 6 h, the particles are collected and washed twice with ethanol and twice with deionized water. The particles are then dried under vacuum at 60 °C for 8 h.

The resulting functionalized magnet with a silica surface can then be utilized to precipitate proteins on it as described in EP 21158984.1 while being used to mix a solution containing said proteins on a device as described in EP 19733052.5, PCT/EP2021/062677, PCT/EP2021/062680, and PCT/EP2021/062681.

Magnets with silica surface can further be functionalized utilizing art-established reactions like tosylation. The particles are transferred into 10 mL of 1,2-dichlorethane as solvent, a catalytic amount of CoCl₂ · 6 H₂O (5 mol%) and 1 equivalent of p-toluenesulfonic acid is added and stirred under reflux at around 80 °C for 1 hour. The resulting particle can then again be further functionalized as the tosyl group is well-known to be a very good leaving group and therefore an excellent reactive group for any kind of substitution reactions.

### Example 2

### Use of a functionalized magnet for proteolytic digestion

To prove functionality, a tosyl modified particle is washed three times with 600 µl methanol, two times with 1x PBS and two times with coupling buffer (1x PBS, 3 m ammonium sulfate, pH 7.4).

One iST-DIGEST tube (from PreOmics GmbH, Planegg, Munich - Germany) is resuspended in 50 µl ultrapure water.

The washed particle is resuspended in 200 µl Coupling Buffer and the 50 µl DIGEST solution are added. The empty DIGEST tube is rinsed with 50 µl coupling buffer, and the rinsing solution is added to the beads to get a total volume of 300 µl.

The mixture is incubated for 24 h at room temperature on a rotator.

After incubation, the supernatant is removed. The particle is washed three times with 600 µl washing buffer. After washing with 600 µl blocking buffer (PBS, 5 % glycine, pH 7.4), blocking is performed for 2.5 hours in 300 µl blocking buffer at room temperature on a rotator.

The solution is replaced with 190 µl lyophilisation buffer (50 mM MES, pH 6.0) to get an approx. 200 µl volume after washing twice with 600 lyophilisation buffer. The resulting tube with the particle inside is frozen in -20 °C freezer overnight with lyophilisation on the next day. After lyophilisation, the particle is stored at room temperature overnight and then used for a standard digestion test. This test is performed following the standard iST protocol as described by the supplier (PreOmics GmbH, Planegg, Munich - Germany), but by replacing the DIGEST tube by the particle, thereby offering a comparison to the use of the free enzyme mix, i.e. enzymes not coupled to a magnet.

## Claims

1. A solid, magnetized or magnetizable composition comprising
(i) a core consisting of or comprising a ferromagnetic or ferrimagnetic material; and
(ii) at least one surface moiety selected from
(1) a binding moiety;
(2) a biomolecule; and
(3) a chemically reactive moiety;
wherein said moiety is covalently bound to
(a) said core; or
(b) a layer which is directly or indirectly attached to said core.

2. The composition of claim 1, wherein said layer is
(a) the only layer and is directly attached to said core; or
(b) an outermost layer which is indirectly attached to said core and separated therefrom by one or more further layers, wherein adjacent layers are directly attached to each other, and wherein an innermost layer is directly attached to said core.

3. The composition of claim 1 or 2, wherein
(1) said binding moiety is configured for chromatography;
(2) said biomolecule is a protein such as an enzyme; a polypeptide; a peptide; a nucleic acid such as DNA or RNA; or an aptamer; or
(3) said chemically reactive moiety is selected from carboxyl-to-amine reactive groups, amine-reactive groups, sulfhydryl-reactive groups, aldehyde-reactive groups, photo-reactive groups and hydroxyl (non-aqueous)-reactive groups.

4. The composition of claim 1 (b), or claim 2 or 3 referring to claim 1 (b), wherein
(a) in case of one single layer, said single layer; and
(b) in case of a plurality of layers, at least one layer, preferably at least said outermost layer,
consists of or comprises a material which is silica-based, preferably silica, silicates or glass; or is a polymer, preferably comprising styrene, an olefin, an acrylic moiety, an amide and/or a urethane.

5. The composition of claim 1 (b), or claims 2 or 3 referring to claim 1(b), wherein
(a) in case of one single layer, said single layer; and
(b) in case of a plurality of layers, at least one layer, preferably at least said innermost layer,
is a metal-containing layer which consists of or comprises at least a main group metal, a transition group metal, a compound comprising such metal, or an alloy thereof, preferably selected from Au, Zn, Zn chromate, Cu, Ni, Cr, Sn, Ti, TiN and/or Ag.

6. The composition of any one of claims 1 to 5, wherein the outermost layer is porous.

7. A method of producing a solid magnetized composition, said method comprising:
(a) providing a core consisting of or comprising a ferromagnetic or ferrimagnetic material, said core optionally having one or more layers as defined in any one of claims 1 to 9 attached thereto;
(b) covalently attaching at least one surface moiety as defined in claim 1 or 3 to the surface of said core of (a); and
(c) thereafter magnetizing the result obtained in step (b);
thereby obtaining said solid magnetized composition.

8. The method of claim 7, wherein said method further comprises a step of attaching at least one of said one or more layers as defined in any one of claims 1 to 9, wherein said further step is to be effected prior to step (b).

9. The method of claim 7 or 8, wherein step (b) is performed repeatedly.

10. The method of any one of claims 7 to 9, wherein said surface moiety is a first chemically reactive moiety, and wherein step (b) is repeated with a second chemically reactive moiety which is capable of reacting with said first chemically reactive moiety, thereby forming a covalent bond.

11. The method of claim 10, wherein said second chemically reactive moiety comprises a further reactive group, wherein said further reactive group is capable of reacting with another reactive group of a further chemically reactive moiety.

12. The composition of any one of claims 1 to 6, or the method of any one of claims 7 to 11, wherein said ferromagnetic or ferrimagnetic material is selected from
(i) compounds or alloys comprising a rare earth metal such as Nd or Sm; Fe; Co; and/or Ni; said compounds or alloys preferably being selected from NdFeB, SmCo, bismanol, AINiCo and ferrites such as Fe₂O₃ and Fe₃O₄; and
(ii) plastic magnetic material, preferably PANiCNQ.

13. A method of enriching or purifying an analyte comprised in a sample, said method comprising:
(a) bringing a solid magnetized composition as defined in in any one of claims 1 to 6 or 12 into contact with said sample,
a. under conditions which allow binding of said analyte to said surface moiety, wherein said surface moiety is a binding moiety; and/or
b. optionally changing the physicochemical conditions of said sample;
(b) separating the composition with said analyte bound thereto from the remainder of said sample, wherein said separating is effected by magnetic means; and
(c) optionally eluting the bound analyte from the composition.

14. Use of a solid magnetized composition as defined in any one of claims 1 to 6 or 12 for analysis and/or purification of a sample and/or for precipitating an analyte on said composition.

15. Use of a solid magnetized composition as defined in any one of claims 1 to 6 or 12, wherein said surface moiety is a chemically reactive moiety, in solid phase synthesis, preferably of peptides, polypeptides or nucleic acids.

16. Use of a solid magnetized composition as defined in any one of claims 1 to 6 or 12, wherein said surface moiety is a biomolecule, and wherein
(i) said biomolecule is a binding molecule, for separating a cognate binding partner of said binding molecule from a sample comprising said binding partner; or
(ii) said biomolecule is an enzyme, for converting a substrate comprised in a sample into a product.

17. The method of claim 13 or the use of any of claims 14 to 16, wherein said method or said use furthermore comprises
(a) mixing;
(b) lysis of biological cells and/or viruses if present; and/or
(c) fragmenting biomolecules such as proteins or nucleic acids if present,
wherein any of (a) to (c) is effected by exposing said solid magnetized composition to a fluctuating magnetic field.
